Europäisches Patentamt

European Patent Office

Office européen des brevets

(11) Veröffentlichungsnummer: **0 257 336**
**A2**

(12) **EUROPÄISCHE PATENTANMELDUNG**

(21) Anmeldenummer: **87111034.2**

(22) Anmeldetag: **30.07.87**

(51) Int. Cl.⁴: **A61K 7/48 , A61K 9/12**

(30) Priorität: **22.08.86 DE 3628531**

(43) Veröffentlichungstag der Anmeldung:
**02.03.88 Patentblatt 88/09**

(84) Benannte Vertragsstaaten:
**AT BE DE FR GB IT LU NL**

(71) Anmelder: **Merz & Co. GmbH & Co.**
**Eckenheimer Landstrasse 100-104**
**D-6000 Frankfurt/M. 1(DE)**

(72) Erfinder: **Beutler, Rolf, Dr. Dipl.-Chem.**
**Frauensteinstrasse 3**
**D-6000 Frankfurt/M 1(DE)**
Erfinder: **Angst, Gunhild**
**Frankfurter Strasse 54**
**D-6277 Bad Camberg(DE)**
Erfinder: **Wimmer, Thomas, Dr**
**Westerbachstrasse 3**
**D-6000 Frankfurt-M. 90(DE)**

(74) Vertreter: **Wolff, Hans Joachim, Dr.jur.**
**Dipl.-Chem. et al**
**Beil, Wolff & Beil Rechtsanwälte Postfach 80**
**01 40 Adelonstrasse 58**
**D-6230 Frankfurt am Main 80(DE)**

(54) **Verschäumbare cremes.**

(57) Verschäumbare Cremes zur Anwendung auf der Haut, bestehend aus einer Creme-Emulsion und einem Treibmittel. Verbesserung der Eigenschaften des Schaumes durch bestimmte Zusammensetzung und Viskosität der Creme-Emulsion.

EP 0 257 336 A2

## Verschäumbare Cremes

Die Erfindung betrifft aus einem Spender-Flakon mittels Treibmitteln verschäumbare Cremes zur Anwendung auf der Haut.

Die Anforderungen an solche verschäumbare Cremes oder Lotionen sind insbesondere eine genügend grosse Schaumstabilität, lockere und sahnige Konsistenz, glänzendes nicht-mattes Aussehen sowie gute Verteilbarkeit und leichte Aufnahme durch die Haut. In der Praxis hat es bisher immer Schwierigkeiten bereitet, all diese Eigenschaften in einem Produkt zu vereinigen, ohne für einen Vorteil auf einen anderen verzichten zu müssen. Es ist bereits versucht worden, diesem Problem dadurch beizukommen, dass man als Treibmittel bestimmte Gase in einem bestimmten Gewichtsverhältnis zueinander vorgeschlagen hat (DE-PS 24 06 109).

Es wurde jedoch jetzt gefunden, dass die Eigenschaften solcher Schäume deutlich verbessert werden können, insbesondere ein glänzender, sahniger Schaum mit Stabilität innerhalb mindestens 5 Minuten erhalten werden kann, wenn - unabhängig vom Treibmittel - die in den Spender-Flakon einzufüllende und mit dem Treibmittel zu versetzende Creme-Emulsion bestimmte Zusammensetzungen und bestimmte physikalische Parameter aufweist. Dabei werden die Verbesserungen gegenüber beliebigen Treibmitteln erzielt, jedoch können durch die Wahl des Treibmittels weitere graduelle Verbesserungen bewirkt werden.

Die erfindungsgemässe Emulsion enthält 2,0-9,0 Gew.-% nicht-ionische Netzmittel, 0,5 - 4,5 Gew.-% Konsistenzgeber, 4,5 - 21,0 Gew.-% Ölanteile, Rest ad 100 Gew.-% Wasser und hat eine Viskosität von 200 - 500 mPas gemessen mit dem Gerät ROTOVISCO der Fa.Haake, System MVI, Messkorf 500, bei 64 U/Min. und 25°C.

Als nicht-ionische Netzmittel können z.B. verwendet werden Alkylpolyglykolphosphorsäureester, Glycerylstearat, PEG-Glycerylstearat, PEG-Stearat, PEG-Stearylether und PEG-Cetylstearylether, und zwar jedes für sich alleine oder in Mischung miteinander.

Geeignete Konsistenzgeber sind makromolekulare Gerüstbildner, Fette, Wachse und Alkohole langkettiger Fettsäuren.

Der Ölanteil wird gewählt aus dem Bereich der auffettenden Stoffe wie pflanzliche und mineralische Öle, flüssige Fettalkohole und flüssige Wachse.

Neben der Wahl der Emulsionskomponenten und der Einhaltung ihrer Mengenbereiche ist es erfingungsgemäss auch wichtig, die obigen Viskositäten einzustellen, d.h. die Viskosität der noch nicht mit dem Treibmittel versetzten Creme-Emulsion. Achtet man nicht gleichzeitig auf die Einhaltung der Viskositätswerte, so kann es trotz Einhaltung der erfindungegemässen Mengenbereiche der Emulsion zu nicht ganz befriedigenden Eigenschaften der verschäumten Creme kommen. Es liegt auf der Hand, dass die Viskosität der Emulsion durch ihre Zusammensetzung, d.h. durch ihre Bestandteile und deren Mengenverhältnisse, bestimmt wird. Sollte sich also bei einer innerhalb der erfindungsgemässen Grenzen angefertigten Emulsion herausstellen, dass sie die erfingungsgemässen Viskositätswerte nicht aufweist, so sind - im erfindungsgemässen Rahmen -die Mengen der Bestandteile entsprechend zu verändern.

Die Viskosität der Emulsion beeinflusst zusammen mit der Art des Treibmittels insbesondere die Schaumdichte und die Blasengrösse des Creme-Schaumes. Angestrebt wird eine Schaumdichte von 0,100 - 0,160 g/cm³ und eine durchschnittliche Blasengrösse von 4 - 20 μm.

Das besonders bevorzugte Treibmittel ist Lachgas ($N_2O$). Es können jedoch auch andere übliche Treibmittel, wie z.B. Kohlendioxid oder Mischungen aus Lachgas mit Kohlendioxid, mit Stickstoff oder mit Fluorkohlenwasserstoffen verwendet werden. Im Falle der Verwendung von Treibmittelmischungen werden folgende Gewichtsverhältnisse bevorzugt:

Lachgas : Kohlendioxid = 1 : 0,5 - 1
Lachgas : Fluorkohlenwasserstoffe = 1 : 0,8 - 0,9
Lachgas : Stickstoff = 1 : 0,5 - 1

Die Erfindung wird anhand der nachfolgenden Beispiele näher erläutert.

## Beispiel 1:

1. Rezepturbestandteile:

| | | |
|---|---|---|
| Nichtionische Netzmittel: | Alkylpolyglykolphosphorsäureester | 2,0 Gew.-% |
| | Polyglyceryl-2-PEG-4-stearat | 6,0 Gew.-% |
| | PEG-12-cetylstearylether | 1,0 Gew.-% |
| Ölanteil: | Paraffinöl | 6,0 Gew.-% |
| | Avocadoöl | 1,0 Gew.-% |
| | Isopropylpalmitat | 5,0 Gew.-% |
| | Jojobaöl | 1,0 Gew.-% |
| | Octyldodecanol | 3,0 Gew.-% |
| Konsistenzgeber: | Cetylstearylalkohol | 2,0 Gew.-% |
| | Carboxyvinylpolymer, neutral. | 0,7 Gew.-% |
| Wässrige Phase: | Wasser | ad 100,0 Gew.-% |

2. Viskosität:     340 mPas

3. Treibmittel:     $N_2O$

4. Schaumdichte:     $0,1306 \ g/cm^3$

5. Durchschnittl. Blasengrösse:     $10 \ \mu m$

6. Stabilität:     $>10$ Min.

Glanz:     glänzend

Konsistenz:     sahnig

### Beispiel 2a:

1. Rezepturbestandteile:

| | | |
|---|---|---|
| Nichtionische Netzmittel: | PEG-2-stearylether | 4,5 Gew.-% |
| | PEG-10-stearylether | 1,5 Gew.-% |
| Ölanteile: | Paraffinöl | 7,0 Gew.-% |
| | Isopropylpalmitat | 7,0 Gew.-% |
| | Octyldodecanol | 6,5 Gew.-% |
| Konsistenzgeber: | Cetylalkohol | 0,5 Gew.-% |
| Wässrige Phase: | Wasser | ad 100,0 Gew.-% |

2. Viskosität: 435 mPas
3. Treibmittel: $N_2O$
4. Schaumdichte: 0,1514 $g/cm^3$
5. Durchschnittl. Blasengrösse: 15 $\mu$m
6. Stabilität: $>$ 10 Min.

Glanz: glänzend

Konsistenz: sahnig

0 257 336

## Beispiel 2b:

1. Rezepturbestandteile:
   Nichtionische Netzmittel: PEG-2-stearylether 4,5 Gew.-%
   
   PEG-10-stearylether 1,5 Gew.-%
   
   Ölanteile: Paraffinöl 7,0 Gew.-%
   
   Isopropylpalmitat 7,0 Gew.-%
   
   Octyldodecanol 6,5 Gew.-%
   
   Konsistenzgeber: Cetylalkohol 0,5 Gew.-%
   Wässrige Phase: Wasser ad 100,0 Gew.-%
2. Viskosität: 435 mPas
3. Treibmittel: $CO_2$
4. Schaumdichte: $0,1523$ g/cm$^3$
5. Durchschnittl. Blasengrösse: 15 $\mu$m
6. Stabilität: $>$ 10 Min.
Glanz: glänzend
Konsistenz: sahnig

0 257 336

## Beispiel 3:

1. Rezepturbestandteile:
   Nichtionische Netzmittel:

   PEG-20-Glycerylstearat 3,0 Gew.-%

   Glycerylstearat 3,0 Gew.-%

   Ölanteile: Paraffinöl 8,0 Gew.-%

   Isopropylpalmitat 8,0 Gew.-%

   Octyldodecanol 3,7 Gew.-%

   Konsistenzgeber: Cetylstearylalkohol 1,3 Gew.-%

   Carboxyvinylpolymer, neutral. 0,7 Gew.-%

   Wässrige Phase: Wasser ad 100,0 Gew.-%

2. Viskosität: 340 mPas

3. Treibmittel: $N_2O$

4. Schaumdichte: $0,1444 \ g/cm^3$

5. Durchschnittl. Blasengrösse: 10 $\mu$m

6. Stabilität: > 10 Min.

Glanz: glänzend

Konsistenz: sahnig

0 257 336

## Beispiel 4a:

1. Rezepturbestandteile:

| | | | |
|---|---|---|---|
| Nichtionische Netzmittel: | Alkylpolyglykolphosphorsäureester | | 2,0 Gew.-% |
| | Polyglyceryl-2-PEG-4-stearat | | 6,0 Gew.-% |
| | PEG-12-cetylstearylether | | 1,0 Gew.-% |
| Ölanteile: | Paraffinöl | | 3,0 Gew.-% |
| | Avocadoöl | | 1,0 Gew.-% |
| | Isopropylpalmitat | | 2,0 Gew.-% |
| | Jojobaöl | | 1,0 Gew.-% |
| | Octyldodecanol | | 2,0 Gew.-% |
| Konsistenzgeber: | Cetylstearylalkohol | | 2,0 Gew.-% |
| | Carboxyvinylpolymer, neutral. | | 0,7 Gew.-% |
| Wässrige Phase: | Wasser | ad 100,0 Gew.-% |

2. Viskosität:    295 mPas

3. Treibmittel:    $N_2O$

4. Schaumdichte:    0,1280 $g/cm^3$

5. Durchschnittl. Blasengrösse:    10 µm

6. Stabilität:    >10 Min.

Glanz:    glänzend

Konsistenz:    sahnig

## Beispiel 4b:

1. Rezepturbestandteile:

| | | |
|---|---|---|
| Nichtionische Netzmittel: | Alkylpolyglykolphosphorsäureester | 2,0 Gew.-% |
| | Polyglyceryl-2-PEG-4-stearat | 6,0 Gew.-% |
| | PEG-12-cetylstearylether | 1,0 Gew.-% |
| Ölanteile: | Paraffinöl | 3,0 Gew.-% |
| | Avocadoöl | 1,0 Gew.-% |
| | Isopropylpalmitat | 2,0 Gew.-% |
| | Jojobaöl | 1,0 Gew.-% |
| | Octyldodecanol | 2,0 Gew.-% |
| Konsistenzgeber: | Cetylstearylalkohol | 2,0 Gew.-% |
| | Carboxyvinylpolymer, neutral. | 0,7 Gew.-% |
| Wässrige Phase: | Wasser | ad 100,0 Gew.-% |

2. Viskosität: 295 mPas

3. Treibmittel: $CO_2$

4. Schaumdichte: 0,1523 g/cm$^3$

5. Durchschnittl. Blasengrösse: 10 $\mu$m

6. Stabilität: $>$10 Min.

Glanz: glänzend

Konsistenz: sahnig

0 257 336

**Beispiel 5a:**

1. Rezepturbestandteile:
   Nichtionische Netzmittel:        PEG-2-stearylether        4,5 Gew.-%

   PEG-10-stearylether      .1,5 Gew.-%

   Ölanteile:        Paraffinöl        6,5 Gew.-%

   Isopropylpalmitat      5,0 Gew.-%

   Octyldodecanol      6,0 Gew.-%

   Avocadoöl      1,0 Gew.-%

   Jojobaöl      1,0 Gew.-%

   Konsistenzgeber:      Cetylalkohol      1,5 Gew.-%

   Wässrige Phase:      Wasser      ad 100,0 Gew.-%

2. Viskosität:      560 mPas

3. Treibmittel:      $N_2O$

4. Schaumdichte:      0,1695 g/cm$^3$

5. Durchschnittl. Blasengrösse:      10 $\mu$m

6. Stabilität:      >10 Min.

Glanz:      glänzend

Konsistenz:      steif

0 257 336

**Beispiel 5b:**

1. Rezepturbestandteile:

   Nichtionische Netzmittel:     PEG-2-stearylether     4,5 Gew.-%

       PEG-10-stearylether     1,5 Gew.-%

   Ölanteile:     Paraffinöl     6,5 Gew.-%

       Isopropylpalmitat     5,0 Gew.-%

       Octyldodecanol     6,0 Gew.-%

       Avocadoöl     1,0 Gew.-%

       Jojobaöl     1,0 Gew.-%

   Konsistenzgeber:     Cetylalkohol     1,5 Gew.-%

   Wässrige Phase:     Wasser     ad 100,0 Gew.-%

2. Viskosität:     560 mPas

3. Treibmittel:     $CO_2$

4. Schaumdichte:     0,2480 g/cm$^3$

5. Durchschnittl. Blasengrösse:     15 $\mu$m

6. Stabilität:     >10 Min.

Glanz:     glänzend

Konsistenz:     steif

0 257 336

## Beispiel 6a:

1. Rezepturbestandteile:

| | | |
|---|---|---|
| Anionische Netzmittel: | Triethanolaminstearat | 5,0 Gew.-% |
| | Natriumlaurylsulfat | 0,25 Gew.-% |
| Nichtionisches Netzmittel: | Sorbitanmonolaurat | 0,5 Gew.-% |
| | Lanolinalkohol | 2,5 Gew.-% |
| Ölanteile: | Isopropylmyristat | 0,5 Gew.-% |
| Konsistenzgeber: | Cetylalkohol | 1,0 Gew.-% |
| | Talkum | 2,0 Gew.-% |
| Wässrige Phase: | Wasser | ad 100,0 Gew.-% |

2. Viskosität: 420 mPas

3. Treibmittel: $N_2O$

4. Schaumdichte: $0,1817 \text{ g/cm}^3$

5. Durchschnittl. Blasengrösse: $> 20 \mu\text{m}$

6. Stabilität: 2 Minuten

Glanz: glänzend

Konsistenz: zu flüssig

0 257 336

## Beispiel 6b:

1. Rezepturbestandteile:

| | | |
|---|---|---|
| Anionische Netzmittel: | Triethanolaminstearat | 5,0 Gew.-% |
| | Natriumlaurylsulfat | 0,25 Gew.-% |
| Nichtionisches Netzmittel: | Sorbitanmonolaurat | 0,5 Gew.-% |
| | Lanolinalkohol | 2,5 Gew.-% |
| Ölanteile: | Isopropylmyristat | 0,5 Gew.-% |
| Konsistenzgeber: | Cetylalkohol | 1,0 Gew.-% |
| | Talkum | 2,0 Gew.-% |
| Wässrige Phase: | Wasser | ad 100,0 Gew.-% |

2. Viskosität:     420 mPas

3. Treibmittel:     $CO_2$

4. Schaumdichte:     0,3020 g/cm$^3$

5. Durchschnittl. Blasengrösse:     > 20 $\mu$m

6. Stabilität:     3 Minuten

Glanz:     glänzend

Konsistenz:     zu flüssig

0 257 336

## Beispiel 7:

1. Rezepturbestandteile:

| | | |
|---|---|---|
| Anionische Netzmittel: | Triethanolaminstearat | 5,0 Gew.-% |
| | Natriumlaurylsulfat | 0,25 Gew.-% |
| Nichtionisches Netzmittel: | Sorbitanstearat | 0,5 Gew.-% |
| | Lanolinalkohol | 5,0 Gew.-% |
| Ölanteile: | Isopropylmyristat | 10,0 Gew.-% |
| Konsistenzgeber: | Cetylalkohol | 0,5 Gew.-% |
| Wässrige Phase: | Wasser | ad 100,0 Gew.-% |

2. Viskosität: 900 mPas

3. Treibmittel: $N_2O$

4. Schaumdichte: 0,1866 g/cm$^3$

5. Durchschnittl. Blasengrösse: $>$ 20 $\mu$m

6. Stabilität: 2 Minuten

Glanz: glänzend

Konsistenz: zu flüssig

0 257 336

## Beispiel 8a:

1. Rezepturbestandteile:
   Nichtionische Netzmittel:  PEG-12-cetylstearylether  . 2,0 Gew.-%
   Ölanteile:  Paraffinöl  7,0 Gew.-%
   Isopropylpalmitat  7,4 Gew.-%
   Octyldodecanol  7,4 Gew.-%
   Konsistenzgeber:  Stearinsäure  3,5 Gew.-%
   Cetylstearylalkohol  0,7 Gew.-%
   Wässrige Phase:  Wasser  ad 100,0 Gew.-%
2. Viskosität:  110 mPas
3. Treibmittel:  $N_2O$
4. Schaumdichte:  $0,1204 \ g/cm^3$
5. Durchschnittl. Blasengrösse:  15 μm
6. Stabilität:  1 Minute
   Glanz:  glänzend
   Konsistenz:  zu flüssig

0 257 336

**Beispiel 8b:**

1. Rezepturbestandteile:

| | | |
|---|---|---|
| Nichtionische Netzmittel: | PEG-12-cetylstearylether | 2,0 Gew.-% |
| Ölanteile: | Paraffinöl | 7,0 Gew.-% |
| | Isopropylpalmitat | 7,4 Gew.-% |
| | Octyldodecanol | 7,4 Gew.-% |
| Konsistenzgeber: | Stearinsäure | 3,5 Gew.-% |
| | Cetylstearylalkohol | 0,7 Gew.-% |
| Wässrige Phase: | Wasser | ad 100,0 Gew.-% |

2. Viskosität: 110 mPas

3. Treibmittel: $CO_2$

4. Schaumdichte: 0,1673 g/cm$^3$

5. Durchschnittl. Blasengrösse: 15 µm

6. Stabilität: 0,5 Minuten

Glanz: glänzend

Konsistenz: zu flüssig

0 257 336

## Beispiel 9:

1. Rezepturbestandteile:

| | | |
|---|---|---|
| Nichtionisches Netzmittel: | Polyglyceryl-2-PEG-4-stearat | 2,0 Gew.-% |
| Kationisches Netzmittel: | Distearyldimethylammoniumchlorid | 1,0 Gew.-% |
| Ölanteile: | Paraffinöl | 7,0 Gew.-% |
| | Octyldodecanol | 8,0 Gew.-% |
| | Isopropylpalmitat | 7,5 Gew.-% |
| Konsistenzgeber: | Cetylalkohol | 0,5 Gew.-% |
| Wässrige Phase: | Wasser | ad 100,0 Gew.-% |

2. Viskosität: 160 mPas

3. Treibmittel: $N_2O$

4. Schaumdichte: 0,1773 g/cm$^3$

5. Durchschnittl. Blasengrösse: 15 µm

6. Stabilität: > 10 Min.

Glanz: glänzend

Konsistenz: steif

0 257 336

**Beispiel 10a:**

1. Rezepturbestandteile:

| | | |
|---|---|---|
| Nichtionische Netzmittel: | Glycerinstearat | 5,0 Gew.-% |
| | PEG-12-cetylstearatether | 3,0 Gew.-% |
| Ölanteile: | Paraffinöl | 7,0 Gew.-% |
| | Isopropylpalmitat | 7,0 Gew.-% |
| | Octyldodecanol | 4,2 Gew.-% |
| Konsistenzgeber: | Cetylstearylalkohol | 0,8 Gew.-% |
| Wässrige Phase: | Wasser | ad 100,0 Gew.-% |

2. Viskosität: 130 mPas

3. Treibmittel: $N_2O$

4. Schaumdichte: $0,1163 \ g/cm^3$

5. Durchschnittl. Blasengrösse: > 20 $\mu$m

6. Stabilität: 2 Minuten

Glanz: glänzend

Konsistenz: zu flüssig

0 257 336

## Beispiel 10b:

1. Rezepturbestandteile:

| | | |
|---|---|---|
| Nichtionische Netzmittel: | Glycerinstearat | 5,0 Gew.-% |
| | PEG-12-cetylstearatether | 3,0 Gew.-% |
| Ölanteile: | Paraffinöl | 7,0 Gew.-% |
| | Isopropylpalmitat | 7,0 Gew.-% |
| | Octyldodecanol | 4,2 Gew.-% |
| Konsistenzgeber: | Cetylstearylalkohol | 0,8 Gew.-% |
| Wässrige Phase: | Wasser | ad 100,0 Gew.-% |

2. Viskosität:  130 mPas

3. Treibmittel:  $CO_2$

4. Schaumdichte:  $0,1570 \text{ g/cm}^3$

5. Durchschnittl. Blasengrösse:  15 μm

6. Stabilität:  2 Minuten

Glanz:  glänzend

Konsistenz:  zu flüssig

0 257 336

**Beispiel 11:**

1. Rezepturbestandteile:
   Nichtionisches Netzmittel:     Polyglykolstearinsäureester     5,0 Gew.-%
   Ölanteile:                     Paraffinöl                      7,0 Gew.-%
                                  Isopropylpalmitat               7,0 Gew.-%
                                  Octyldodecanol                  7,0 Gew.-%
   Konsistenzgeber:               Cetylalkohol                    1,0 Gew.-%
   Wässrige Phase:                Wasser                     ad 100,0 Gew.-%
2. Viskosität:                    70 mPas
3. Treibmittel:                   $N_2O$
4. Schaumdichte:                  0,1429 g/cm$^3$
5. Durchschnittl. Blasengrösse:   > 20 µm
6. Stabilität:                    1 Minute
Glanz:                            glänzend
Konsistenz:                       zu flüssig

0 257 336

Diskussion der Beispiele:

In allen Beispielen, die mit a und b gekennzeichnet sind, hat b dieselbe Zusammensetzung der Emulsion wie a, verwendet jedoch als Treibmittel Kohlendioxid anstatt Lachgas.

Die Beispiele 1 - 4 beschreiben Emulsionen mit erfindungsgemässen Zusammensetzungen und Viskositäten.

Ein Vergleich der Beispiele 6, 7, 8, 9 und 11 mit den erfindungsgemässen Beispielen 1 - 4 zeigt, dass die Schaumeigenschaften schlechter sind, wenn die Rezeptur der Emulsion nicht eingehalten wird.

Die Beispiele 5 und 10 haben zwar eine erfindungsgemässe Zusammensetzumg der Emulsion, liegen jedoch ausserhalb der erfingungsgemässen Viskositätswerte. Diese Beispiele zeigen also, dass gleichzeitig auch auf die Einhaltung der Viskositätswerte geachtet werden muss. Damit korrespondiert Beispiel 6, wo zwar die Viskosität der Emulsion im erfindungsgemässen Bereich liegt, nicht aber deren Zusammensetzung.

Darüberhinaus zeigen die Beispiele 6 und 7, dass es auch auf die Verwendung ausschliesslich nichtionischer Netzmittel ankommt. In Beispiel 6 liegt die Summe der Netzmittel zwar im erfindungsgemässen Bereich, jedoch werden dort neben nicht-ionischen auch anionische Netzmittel verwendet. In Beispiel 7 liegt die Menge der nichtionischen Netzmittel zwar im erfindungsgemässen Bereich jedoch werden dort darüberhinaus auch anionische Netzmittel eingesetzt.

Der Vergleich der jeweiligen Beispiele a und b untereinander zeigt schiesslich, dass die Auswechslung der Treibmittel keinen entscheidenden Einfluss auf die erfindungsgemässen Eigenschaften des Schaumes hat. Ist die Emulsion bezüglich Zusammensetzung und Viskosität erfindungsgemäss, bleiben die Eigenschaften des Schaumes auch bei Verwendung eines anderen Treibmittels gut und umgekehrt erhalten sie durch die Abwandlung des Treibmittels nicht die gewünschten Eigenschaften, wenn die Emulsion den erfindungsgemässen Anforderungen nicht entspricht.

## Ansprüche

1. Verschäumbare Cremes aus einer Creme-Emulsion und einem Treibmittel, dadurch gekennzeichnet, dass die Creme-Emulsion 2,0 - 9,0 Gew.-% nicht-ionische Netzmittel, 0,5 - 4,5 Gew.-% Konsistenzgeber, 4,5 - 21,0 Gew.-% Ölanteile und Wasser ad 100,0 Gew.-% enthält und eine Viskosität von 200 - 500 mPas hat.

2. Zusammensetzung nach Anspruch 1, dadurch gekennzeichnet, dass das Treibmittel Lachgas ist.